# EUROPEAN PATENT SPECIFICATION

(11) **EP 4 444 231 B1**
(45) Date of publication and mention of the grant of the patent: **07.10.2026**
(21) Application number: 22836044.2
(22) Date of filing: 09.12.2022
(51) Int. Cl.: A61F 2/68, A61F 2/70

(54) **MULTIMODAL HINGE JOINT SYSTEM FOR PROSTHESIS OR ORTHOSIS**
MULTIMODALES SCHARNIERGELENKSYSTEM FÜR PROTHESEN ODER ORTHESEN
SYSTÈME D'ARTICULATION CHARNIÈRE MULTIMODALE POUR PROTHÈSE OU ORTHÈSE

(30) Priority: 10.12.2021 NL 2030109
(43) Date of publication of application: 16.10.2024
(73) Proprietor: Axiles Bionics BV, 1130 Brussel (BE)
(72) Inventor: CHERELLE, Pierre Philippe, 1472 VIEUX-GENAPPE (BE); GOMEZ, Felipe, 1310 LA HULPE (BE); DE MUYTER, Julien, 1030 SCHAERBEEK (BE); LAUWERS, Gil, 1050 IXELLES (BE)
(74) Representative: Arnold & Siedsma
(86) International application number: PCT/EP2022/085237
(87) International publication number: WO 2023/105067

(56) References cited:
- JP-A- 2018 111 198
- US-A1- 2017 165 088
- US-B2- 10 080 672
- US-B2- 8 900 325

## Description

### FIELD OF INVENTION

The field of the invention relates to multimodal hinge joint systems for prostheses, orthoses, or artificial limbs. Particular embodiments relate to matters pertaining to prostheses or orthoses for functionally assisting, enhancing, and/or replacing a limb of a human or animal subject or for augmenting a body or a part of a body of a human or animal subject.

### BACKGROUND

In existing prostheses or orthoses, as well as when fabricating an artificial limb for a humanoid or animal-inspired robot, reproduction of a joint motion as occurring naturally in humans and animals remains a complex technical challenge. Indeed, while walking for example, humans use a cyclic sequence of limb movements to move the body forward and maintain stance stability. This is accomplished by a process called the double pendulum. And although walking is by far the most basic and common thing in life, it involves very complex mechanisms including energy storing, transfer and return which depend on a highly complex anatomical bone, muscle and tendon structure. The main mechanical challenges lie in accurately transitioning between the different phases of the cyclic sequence provoked by the limb movements for a user of the prosthesis or orthosis to have a more comfortable experience during use, and maintaining the motion dynamic and balance of walking when creating a limb for a humanoid or animal-inspired robot. However, complexity of the mechanism used for transitioning through this cyclic sequence has to be balanced with the weight of the mechanism itself.

US2017/0165088 A1 discloses a prosthesis or orthosis having a movement controlling mechanism (MCM) including a first MCM part, a second MCM part and one or more intermediate elements and biasing mechanism which, in a contacting mode of operation of the MCM, bias the intermediate elements against a MCM part.

In prior art solution, to address the above mentioned problems relative to prostheses or orthoses, transitioning through the cyclic sequence has been oversimplified in hinge joint systems and concerns have mainly been directed to a walking usage on a relatively level surface, leading to uneasiness for the user using a prosthesis or orthosis with such hinge joint systems in more realistic situations. There is thus a need for a prosthesis or orthosis comprising a hinge joint system reproducing more completely the transitions through a desired cyclic sequence as well as being more flexible. This need is replicated in regards to limbs for humanoid or animal-inspired robots.

### SUMMARY

The object of embodiments of the invention is to provide a prosthesis or orthosis comprising a hinge joint system allowing improving the comfort of the prosthesis or orthosis for a user by more completely controlling motions of the hinge joint system, as well as providing a limb comprising the hinge joint system allowing a more faithful and technically reliable imitation of a human joint for humanoid or animal-inspired robots.

According to a first aspect of the invention, there is provided a prosthesis or orthosis comprising a hinge joint system for functionally assisting, enhancing, and/or replacing a hinge joint of a human or animal subject. Alternatively, there is provided a limb comprising the hinge joint system for functionally working as a hinge joint of a humanoid or animal-inspired robot. The hinge joint system comprises: a first member, a second member, a passive elastic element, and a torque controlling mechanism. The second member is pivotally coupled to the first member around a first axis. The passive elastic element is fixed to the second member and acts on the torque, and thus the rotation, around the first axis. The passive elastic element is configured for storing energy when a load is applied to it. The load applied to it may be the result of an applied torsion, elongation, tension, and/or pressure, depending on a working direction of the passive elastic element along which the load is applied and on a type of the passive elastic element.

The torque controlling mechanism is mounted between the first member and the passive elastic element, and is configured for controlling a torque around the first axis. The torque controlling mechanism is configured for controlling the torque around the first axis in at least three modes of operations:
- a first mode wherein load is not transmitted between the first member and the passive elastic element in both working directions of the passive elastic element,
- a second mode wherein load is transmitted between the first member and the passive elastic element in one working direction of the passive elastic element and not transmitted in the other working direction of the passive elastic element,
- a third mode wherein load is transmitted between the first member and the passive elastic element in both working directions of the passive elastic element.

By having at least three modes of operations, the prosthesis or orthosis can adapt to a greater variety of operational conditions. For example, the third mode may be used during rest, or during maintenance, so that there is no unexpected motion, or lack thereof, of the prosthesis or orthosis.

The torque controlling mechanism may comprise additional active or passive elements in order to provide energy for the motion of the first member relative to the second member. Examples of active elements comprise linear or rotary motorized actuators. Examples of passive elements comprise elastic elements storing energy due to the application of a load. In the prosthesis or orthosis, the first member and the second member may correspond to portions of an articulated limb and the hinge joint system to a corresponding joint, *e.g.* an ankle joint.

In the case of a foot prosthesis or orthosis, for example, in the second mode, transmission of the load between the first member and the passive elastic element is possible in a direction corresponding to a dorsiflexion, while transmission of the load is not possible in a direction corresponding to a plantarflexion. By transmission of the load between the first member and the passive elastic element, it is meant that force applied to the first member is directly converted to a load, positive or negative, exercised on the passive elastic element and stored as energy, and inversely, energy stored in the passive elastic element is directly exerted as a load to the first member translating as a force. A force applied to the second member, due to the fixation of the second member with the passive elastic element, will also potentially end in the transmission of load between the first member and the passive elastic element

Then, depending on the three modes of operations, and due to a mounting of the passive elastic element within the mechanical structure of the prosthesis or orthosis in order to act on the rotation around the first axis, and to a mounting of the torque controlling mechanism between the first member and the passive elastic element, a torque will be created between the first member and the second member. This torque may depend, amongst others, on the transmission variance of the modes of operations of the torque controlling mechanism, external forces applied to the first member, external forces applied to the second member, mechanical characteristics of the passive elastic element, and/or other forces applied to the passive elastic element by the torque controlling mechanism.

Since a torque is created between the first member and the second member around the first axis, due to the passive elastic element being fixed to the second member on one hand and to the torque controlling mechanism on the other hand, and to the second member being pivotally coupled with the first member, a rotation of the second member relative to the first member around the firs axis is obtained.

When a force is applied to the second member; while in the second mode, for example, transmission of the load not being allowed in the other working direction is facilitating rotation of the passive elastic element with respect to the first member. This rotation may be caused by the restoring force of the passive elastic element which then acts on the rotation of the second member relative to the first member around the first axis.

Preferably, transitions between the first mode and the second mode are triggered depending on the phase in a gait cycle. For example, the second mode may be used during the stance phase of the gait cycle, and the first mode may be used during the swing phase of the gait cycle. So, thanks to the clutch mechanism, motions of the prosthesis or orthosis are made more comfortable for the user and adaptable.

Additionally, due to the passive elastic element, a load applied by the subject wearing the prosthesis or orthosis may be stored and released, thereby improving the energy efficiency of the prosthesis or orthosis. Indeed, when in the second mode, as long as a load between the first member and the passive elastic element is exerted in the direction in which it is transmitted, a leveraging force, originating from the torque around the firs axis, above the predetermined resistive threshold defined by the passive elastic element enables rotation around the first axis. By increasing said torque, potential energy can be accumulated in the passive elastic element and by again decreasing said torque the stored energy is again released as kinetic energy.

Thusly, an automatic adaptability of the prosthesis or orthosis to different operational conditions, such as walking on slopes or with varying speeds, can be obtained.

The skilled person will understand that the pivot coupling between the first member and the second member around the first axis may be implemented in various manners. In an embodiment, the pivot coupling may be monocentric and achieved by a single axis element coupling the first member and the second member directly; thus, the first axis is a real axis coinciding with the single axis element. In another embodiment, the pivot coupling may be polycentric and achieved by a plurality of axis elements and linkage elements coupling the first member and the second member indirectly; thus, the first axis is a virtual axis depending on the motion between the first member and the second member.

According to a preferred embodiment, the torque controlling mechanism comprises a selecting means configured for selecting one of the at least three modes of operations, preferably a rotatable selecting means. Preferably, the selecting means is configured to transition between the first mode and the second mode, and between the second mode and the third mode.

In this manner, application of torque around the first axis is controllable at will. The selecting means may be used to transition between the different modes at different timings depending on the operational conditions, *e.g*. walking, running, flat surface, incline, and decline.

According to an exemplary embodiment, the torque controlling mechanism further comprises a first motorized actuator configured for actuating the selecting means.

Additionally or alternatively, the torque controlling mechanism further comprises one or more mechanical actuators configured for actuating the selecting means. The one or more mechanical actuators may comprise at least one of: spring, inertia trigger, manual trigger switch, overrunning clutch, end stop.

In this way, the selecting means can be controlled automatically following a control scheme implemented using the first motorized actuator. For example, the prosthesis or orthosis may comprise a microcontroller configured for controlling the first motorized actuator, and a memory for storing a plurality of control schemes depending on the operational conditions. The microcontroller may control the first motorized actuator such that the torque controlling mechanism transitions between the different modes of operation following the predetermined control scheme.

According to a preferred embodiment, the torque controlling mechanism is further configured for controlling the torque around the first axis in at least a fourth mode of operation, in which energy is stored, *e.g.* in at least one spring, to trigger a change from the second mode to the first mode when the stored energy is under a threshold.

For example, the trigger will occur when the remaining torque around the first axis approaches a value of zero. Considering a foot prosthesis or orthosis, the torque controlling mechanism is designed for this trigger to happen when the foot leaves the ground, *i.e.* when the user has completely transferred his weight to the other foot.

In this manner, a "floating" mode of operation is defined between the first mode and the second mode whose transition from the second mode is triggered by the selecting means, and whose transition to the first mode is mechanically conditioned. This fourth mode allows improving the smoothness in the transition from the second mode to the first mode by conditioning the trigger of the transition based on an amount of stored energy, *e.g.* in at least one spring.

According to a selected embodiment, the torque controlling mechanism further comprises a stopper element configured for stopping the load from being applied to the passive elastic element in one working direction.

Thusly, travel in the motion of the passive elastic element in one working direction, in which load transmission is allowed, may be stopped from going over a preset point. The halting means may be embodied by a mechanical stop.

According to an exemplary embodiment, the torque controlling mechanism further comprises a clutch mechanism. The clutch mechanism comprises: a base portion, a rotating member, a one-way bearing, a shifter means, and a coupling mechanism. The rotating member is provided to the base portion, and configured for rotating relative to the base portion around a second axis. The rotating member preferably comprises a cylindrical portion extending along the second axis. The one-way bearing is provided to the rotating member along the second axis, optionally arranged around the cylindrical portion of the rotating member. The shifter means is configured for being translatable along the second axis and for being non-rotatable relative to the base portion. The coupling mechanism is fixed around the one-way bearing, and is configured for having a first position coupled to the shifter means and a second position uncoupled from the shifter means. A rotation of the coupling mechanism around the second axis relative to the base portion is prevented in the first position and is allowed in the second position.

In this way, the different elements of the clutch mechanism are arranged in a concentric manner which ensures an overall compact form factor of the clutch mechanism.

In an embodiment, the second axis may coincide with the first axis and the clutch mechanism influences directly the torque between the first member and the second member at the level of their pivot coupling. In another embodiment, the second axis may be perpendicular to the first axis and a converting means may be provided between the rotating member and one of the first member or the second member to convert a rotation around the second axis to a rotation around the first axis.

According to a preferred embodiment, the selecting means, preferably a rotatable selecting means, is provided to the shifter means and configured for converting a rotation of the selecting means into a translation of the shifter means.

In this manner, selection of the mode of operation of the torque controlling mechanism is achieved directly with a minimum number of parts. This improves the reliability and compactness of the torque controlling mechanism. Preferably, the rotatable selecting means may be rotated around the second axis.

According to an exemplary embodiment, the at least one spring is mounted between the shifter means and the base portion. Preferably, at least three springs are mounted at regular interval around the second axis between the shifter means and the base portion.

In this way, the spring force is exerted mainly along the translation direction of the shifter means, which is more predictable in order to tune the threshold for the energy stored within to be released. At the same time, slight rotation motions of the shifter means around the second axis which could occur during coupling and uncoupling of the shifter means and the coupling mechanism are still allowed. Preferably, the at least one spring comprises at least one helical tension spring.

According to a preferred embodiment, the torque controlling mechanism further comprises a second motorized actuator configured for rotating the rotating member of the clutch mechanism.

In this manner, the rotation of the rotating member can be controlled more precisely. Preferably the second motorized actuator is a linear actuator, more preferably a ball screw actuator.

According to an exemplary embodiment, the clutch mechanism is mounted to the second motorized actuator.

In this manner, the clutch mechanism can influence directly an output of the second motorized actuator.

In an embodiment, the assembly of the clutch mechanism and the second motorized actuator comprises a first pivot coupling between the first member and a stationary element of the second motorized actuator, and a second pivot coupling between the second member and a dynamic element of the second motorized actuator.

According to a preferred embodiment, the coupling mechanism comprises a first set of gear teeth on its periphery, and the shifter means comprises a second set of gear teeth complementary to the first set of gear teeth. Preferably, at least one of the first set or the second set of gear teeth has gear teeth with a sloped face facing the gear teeth of the other one of the first set or the second set of gear teeth such as to promote engagement of the first set and second set of gear teeth when transitioning from the second position to the first position of the coupling mechanism.

In this way, wear and tear between the first set and the second set of gear teeth is reduced and coupling of the shifter means with the coupling mechanism is facilitated during rotation of the coupling mechanism.

In an embodiment, the shifter means is arranged over the coupling mechanism in a coupling state. The first set of gear teeth is provided to an outer surface of the coupling mechanism, and the second set of gear teeth is provided to an inner surface of the shifter means.

According to a preferred embodiment, the clutch mechanism further comprises a blocking means including a first blocking part configured for inter-engaging with a second blocking part, said first blocking part fixed to the shifter means and said second blocking part fixed to the rotating member; wherein the blocking means is further configured for, in an inter-engaged state of the first blocking part and the second blocking part, preventing the rotation of the rotating member around the second axis allowed by the one-way bearing.

In this way, the third mode of operation can be implemented efficiently. Preferably, the first blocking part and the second blocking part are made up by a complementary pair of inter-engaging teeth arranged in a concentric manner around the second axis. More particularly, the shifter means may comprise a circular row of saw teeth and the rotating member may comprise a corresponding row of saw teeth. When transitioning from the first mode to the second mode, the shifter means and the coupling mechanism are configured to couple, thereby allowing rotation around the second axis in a direction dependent on the one-way bearing. When transitioning from the second mode to the third mode, the shifter means and the coupling mechanism are still coupled, but the shifter means further engage with the rotating member at the level of the first and second blocking parts; therefore suppressing rotation freedom around the second axis.

According to an exemplary embodiment, the selecting means comprises a selector ring surrounding the shifter means. The selector ring comprises at least one cut-out portion configured for cooperating with at least one corresponding protruding portion of the shifter means, such that the rotation of the selecting means is converted into the translation of the shifter means by the cooperation of the at least one cut-out portion and the at least one corresponding protruding portion.

In this manner, compactness of the overall clutch mechanism is preserved. The selector ring may extend along the second axis and be centered on the second axis. The at least one corresponding protruding portion may be fixed on an outer surface of the shifter means. The selector ring may be arranged around the shifter means. Depending on the profile of the at least one cut-out portion, the at least one corresponding protruding portion may be pushed into different positions, thus provoking the translation of the shifter means. Each of the at least one cut-out portion may comprise a plurality of areas. The plurality of areas may include transitioning areas and mode areas. The mode areas can correspond to stable positions of the shifter means with respect to the coupling mechanism and/or the rotating member such that the clutch mechanism is in one of the at least three operation modes. The transitioning areas may comprise profiles sized to correspond to the travel distance necessary for the shifter means to translate such that transition is achieved between the first mode and the second mode, the second mode and the third mode, and *vice versa*.

According to a selected embodiment, the at least one corresponding protruding portion comprises at least one rolling ball.

Thusly, friction is limited between the at least one cut-out portion and the at least one corresponding protruding portion. Preferably, the at least one corresponding protruding portion comprises at least three protruding portions located at regular intervals around the second axis. Additionally, the at least three protruding portions may serve for self-centering the shifter means respective to other elements of the clutch mechanism.

According to a preferred embodiment, the torque controlling mechanism is further configured for controlling the motion of the first member with respect to the second member through leverage.

In this way, the torque controlling mechanism is more energy efficient. Preferably, the torque controlling mechanism is pivotally coupled to the first member and the passive elastic element at opposite extremities, respectively. Both pivot coupling may rotate in a co-axial manner relative to the first axis. So, by acting on one end of the passive elastic element, for example, the torque controlling mechanism may control the motion of the second member with respect to the first member by leveraging force around the first axis.

According to an exemplary embodiment, the torque controlling mechanism further comprises a converting means configured for interconnecting the rotating member and one of the first member or the second member, and for converting the rotation of the rotating member around the second axis to a rotation around a third axis and vice versa, said third axis being preferably parallel to the first axis.

In this manner, the torque controlling mechanism can be more freely positioned relative to the rest of the prosthesis or orthosis. Indeed, using converting means, the torque controlling mechanism action may be "delocalized" with respect to the first axis, due to space or weight constraints for example, while still acting on it.

The skilled person will understand that features and advantages of the above described aspects related to prosthesis and orthosis embodiments apply, *mutatis mutandis*, to the below described aspect related to clutch mechanism embodiments.

In an example, there is provided a clutch mechanism. The clutch mechanism comprises: a base portion, a rotating member, a one-way bearing, a shifter means, and a coupling mechanism. The rotating member is provided to the base portion, and configured for rotating relative to the base portion around a main axis. The rotating member preferably comprises a cylindrical portion extending along the main axis. The one-way bearing is provided to the rotating member along the main axis, optionally arranged around the cylindrical portion of the rotating member. The shifter means is configured for being translatable along the main axis and for being substantially non-rotatable relative to the base portion. The coupling mechanism is fixed around the one-way bearing and is configured for having a first position coupled to the shifter means and a second position uncoupled from the shifter means, such that a rotation of the coupling mechanism around the main axis relative to the base portion is prevented in the first position and allowed in the second position.

By arranging the different components of the clutch mechanism in a concentric manner around the main axis, a compact and efficient clutch mechanism is obtained which is suitable for the control of rotation around the main axis in complex operational situations.

According to a preferred embodiment, the clutch mechanism further comprises a selecting means, preferably a rotatable selecting means, provided to the shifter means and configured for converting a rotation of the selecting means into a translation of the shifter means such as to change the shifter means from the first position to the second position, and *vice versa*, of the coupling mechanism.

According to an exemplary embodiment, the selecting means comprises a selector ring surrounding the shifter means. The selector ring comprises at least one cut-out portion configured for cooperating with at least one protruding portion of the shifter means, such that the rotation of the selecting means is converted into the translation of the shifter means by the cooperation of the at least one cut-out portion and the at least one corresponding protruding portion.

According to a preferred embodiment, the clutch mechanism further comprises at least one spring mounted between the shifter means and the base portion, said at least one spring in which energy is stored to trigger a change from the second position to the first position when the stored energy is under a threshold.

According to an exemplary embodiment, the coupling mechanism comprises a first set of gear teeth on its periphery, and the shifter means comprises a second set of gear teeth complementary to the first set of gear teeth. At least one of the first set or the second set of gear teeth has gear teeth with a sloped face facing the gear teeth of the other one of the first set or the second set of gear teeth such as to promote engagement of the first set and second set of gear teeth when transitioning from the second position to the first position of the coupling mechanism.

According to a preferred embodiment, the clutch mechanism further comprises a blocking means including a first blocking part configured for inter-engaging with a second blocking part, said first blocking part fixed to the shifter means and said second blocking part fixed to the rotating member; wherein the blocking means is further configured for, in an inter-engaged state of the first blocking part and the second blocking part, preventing the rotation of the rotating member around the main axis allowed by the one-way bearing.

According to another embodiment, there is provided a prosthesis or orthosis comprising a hinge joint system for functionally assisting, enhancing, and/or replacing a hinge joint of a human or animal subject. The hinge joint system of the prosthesis or orthosis comprises: a first member, a second member, and a torque controlling mechanism. The second member is pivotally coupled to the first member around a first axis. The torque controlling mechanism is mounted between the first member and the passive elastic element, and is configured for controlling a torque around the first axis. The torque controlling mechanism comprises a clutch mechanism according to the features and aspects of clutch mechanism embodiments described relative to the second aspect of the invention. The clutch mechanism is provided to the prosthesis or orthosis such that the main axis of the clutch mechanism is substantially perpendicular to the first axis.

The skilled person will understand that features and advantages of the above described aspects related to prosthesis and orthosis embodiments and clutch mechanism embodiments apply, *mutatis mutandis*, to the below described aspect related to prosthesis or orthosis embodiments.

In an example, there is provided a prosthesis or orthosis comprising a hinge joint system for functionally assisting, enhancing, and/or replacing a hinge joint of a human or animal subject. Alternatively, there is provided a limb comprising the hinge joint system for functionally working as a hinge joint of a humanoid or animal-inspired robot. The hinge joint system of the prosthesis or orthosis comprises: a first member, a second member, a passive elastic element, and a torque controlling mechanism. The second member is pivotally coupled to the first member around a first axis. The passive elastic element is fixed to the second member and acts on the rotation around the first axis. The passive elastic element is configured for storing energy when a load is applied to it. The torque controlling mechanism is mounted between the first member and the passive elastic element, and is configured for controlling a torque around the first axis. The torque controlling mechanism is configured for controlling the torque around the first axis in at least three modes of operations:
- a first mode,
- a second mode,
- a third mode, in which energy is stored, preferably in at least one spring, to trigger a change from the second mode to the first mode when the stored energy is under a threshold.

The torque controlling mechanism comprises a first static portion and a second dynamic portion. A rotation of the second dynamic portion with respect to the first static component is controlled by at least a third surrounding portion of the torque controlling mechanism around the second dynamic portion. An energy storage means such as at least one spring may be mounted between the first static portion and the third surrounding portion. Preferably, the energy storage means comprises at least three springs mounted at regular intervals around the second dynamic portion.

By arranging an energy storage as a trigger, an automatic trigger is created which can adapt directly, in a more natural way, to the mechanical constraints applied in the transition between the first mode and the second more. Thus, a "floating" mode of operation is defined between the first mode and the second mode which is mechanically conditioned, and which is preferably directly related to the motion of the second member relative to the first member

According to an exemplary embodiment, the first mode and the second mode are any two modes chosen among:
- a free mode wherein load is not transmitted between the first member and the passive elastic element in both working directions of the passive elastic element,
- a one-way mode wherein load is transmitted between the first member and the passive elastic element in one working direction and not transmitted in the other working direction of the passive elastic element,
- a blocked mode wherein load is transmitted between the first member and the passive elastic element in both working directions of the passive elastic element.

The skilled person will understand that features and advantages of the above described aspects related to prosthesis and orthosis embodiments and clutch mechanism embodiments apply, *mutatis mutandis*, to the below described aspect related to prosthesis or orthosis embodiments.

In an example, there is provided a prosthesis or orthosis comprising a hinge joint system for functionally assisting, enhancing, and/or replacing a hinge joint of a human or animal subject. Alternatively, there is provided a limb comprising the hinge joint system for functionally working as a hinge joint of a humanoid or animal-inspired robot. The hinge joint system of the prosthesis or orthosis comprises: a first member, a second member, a passive elastic element, and a torque controlling mechanism. The second member is pivotally coupled to the first member around a first axis. The passive elastic element is fixed to the second member and acts on the rotation around the first axis. The passive elastic element is configured for storing energy when a load is applied to it. The torque controlling mechanism is mounted between the first member and the passive elastic element, and is configured for controlling a torque around the first axis. The torque controlling mechanism is configured for controlling the torque around the first axis in at least two modes of operations:
- a first mode wherein load is not transmitted between the first member and the passive elastic element in both working directions of the passive elastic element,
- a second mode wherein load is transmitted between the first member and the passive elastic element in one working direction of the passive elastic element and not transmitted in the other working direction of the passive elastic element.

The torque controlling mechanism further comprises a coupling gear having a first set of gear teeth, said coupling gear being configured for being engaged to a gear counterpart having a second set of complementary gear teeth, such that engagement of the coupling gear to the gear counterpart corresponds to a transition from the first mode to the second mode. Preferably, at least one of the first set or the second set of gear teeth has gear teeth with a sloped face facing the gear teeth of the other one of the first set or the second set of gear teeth such as to promote engagement of the first set and second set of gear teeth when transitioning from the first mode to the second mode.

By using sloped teeth, wear and tear between the first set and the second set of gear teeth is reduced and coupling during the transition between the first mode and the second mode is facilitated while the first set and the second set of gear teeth are rotating one with respect to the other. This is especially the case in high torque situations which can occur when human or animal muscles are the source of motion between the first member and the second member.

According to a preferred embodiment, the coupling gear comprises a hollow body, preferably a cylindrical hollow body, having an outer surface. The gear counterpart comprises a hollow body, preferably a cylindrical hollow body, having an inner surface. The first set of gear teeth is provided to the outer surface of the coupling gear, and the second set of gear teeth is provided to the inner surface of the gear counterpart.

### BRIEF DESCRIPTION OF THE DRAWINGS

This and other aspects of the present invention will now be described in more detail, with reference to the appended drawings showing a currently preferred embodiment. Like numbers refer to like features throughout the drawings.
Figure 1 illustrates a side view of an exemplary embodiment of a prosthesis or orthosis;
Figures 2A-2B shows perspective views of an exemplary embodiment of a clutch mechanism;
Figure 3 depicts a perspective view of an exemplary embodiment of a first mode of operation of the clutch mechanism;
Figure 4 depicts a perspective view of an exemplary embodiment of a second mode of operation of the clutch mechanism;
Figure 5 depicts a perspective view of an exemplary embodiment of a third mode of operation of the clutch mechanism;
Figure 6 illustrates a close-up view of another exemplary embodiment of a clutch mechanism;
Figures 7A-7B show schematic structural embodiments of a prosthesis or orthosis.

### DETAILED DESCRIPTION OF EMBODIMENTS

It is to be noted in the following that, for the sake of ease of understanding, the embodiments described below in relation with the drawings have been limited to prosthesis or orthosis embodiments. However, the skilled person will understand that the teachings brought by the below embodiments are equally applicable to embodiments of limbs for humanoid or animal-inspired robots.

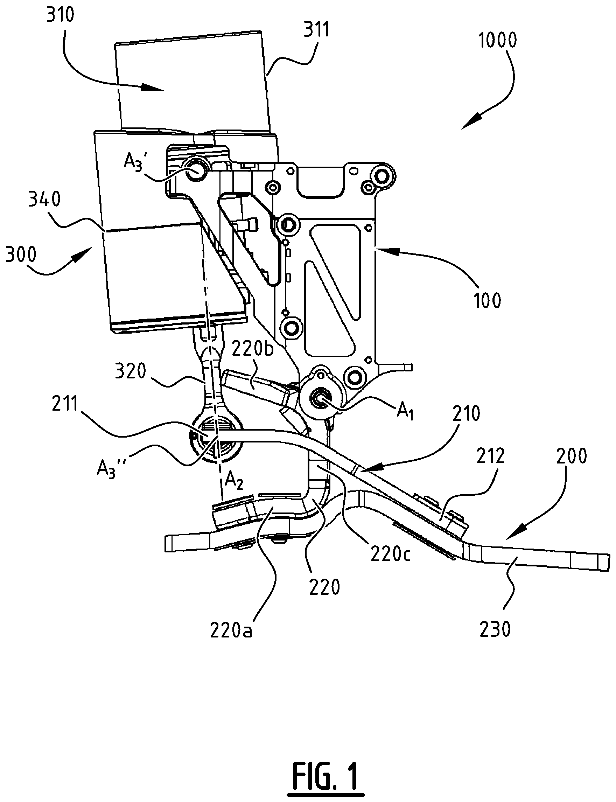

Figure 1 illustrates a side view of an exemplary embodiment of a prosthesis or orthosis. The prosthesis or orthosis 1000 comprises a first member 100, a second member 200, a passive elastic element 210, and a torque controlling mechanism 300. The passive elastic element 210 is fixed to the second member 200.

The torque controlling mechanism 300 is configured for controlling a torque around a first axis A1. The torque controlling mechanism 300 comprises a clutch mechanism 340, and optionally a motorized actuator 310. The clutch mechanism 340 is configured for controlling a rotation around a second axis A2 in order to assist the torque controlling mechanism 300. The torque controlling mechanism 300 is configured for controlling the torque around the first axis A1 in at least two modes of operations:
- a first mode wherein load is not transmitted between the first member 100 and the passive elastic element 210 in both working directions of the passive elastic element 210,
- a second mode wherein load is transmitted between the first member 100 and the passive elastic element 210 in one working direction of the passive elastic element 210 and not transmitted in the other working direction of the passive elastic element 210.

In the embodiment of Figure 1, the torque controlling mechanism 300 is pivotally coupled to the passive elastic element 210 around a second third axis A3". The second third axis A3" is substantially parallel to the first axis A1.

For the sake of simplicity, embodiments related to a foot prosthesis with an ankle joint system for a human subject will be detailed in the following. The skilled person will understand, however, that the disclosed features below can be freely adapted to an animal subject or for another limb, for example.

Briefly, in the case of a foot prosthesis or orthosis, for example, in the second mode, transmission of the load between the first member 100 and the passive elastic element 210 is possible in a direction corresponding to a dorsiflexion, while transmission of the load is not possible in a direction corresponding to a plantarflexion. By transmission of the load between the first member 100 and the passive elastic element 210, it is meant that force applied to the first member 100 is directly converted to a load, positive or negative, exercised on the passive elastic element and stored as energy, and inversely, energy stored in the passive elastic element 210 is directly exerted as a load to the first member 100 translating as a force.

A force applied to the second member 200, due to the fixation of the second member 200 with the passive elastic element 210, will also potentially end in the transmission of load between the first member 100 and the passive elastic element 210.

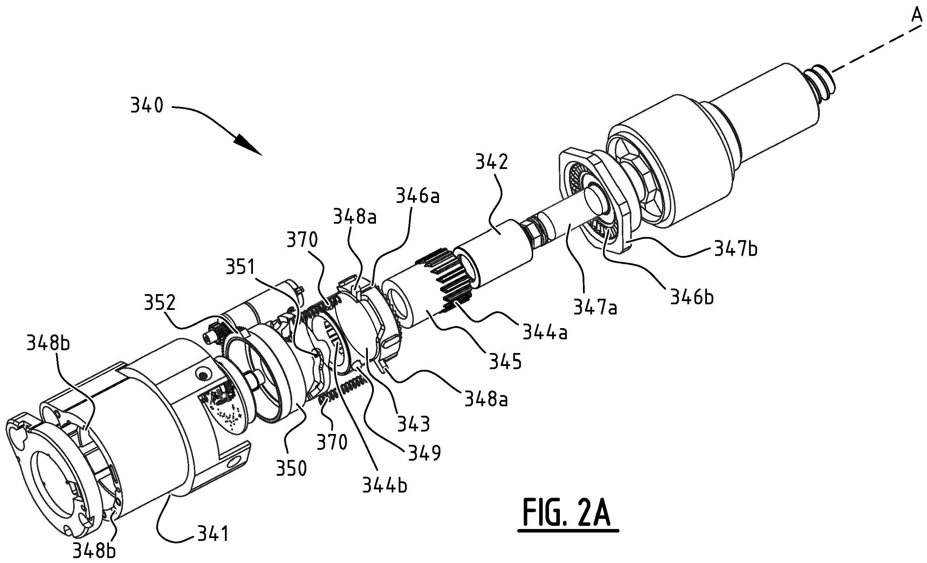

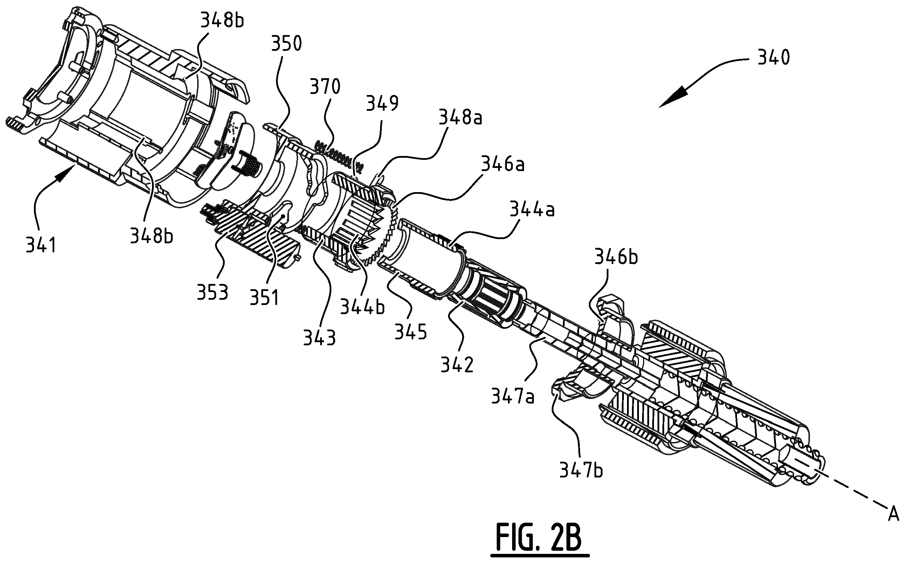

Transitions between the first mode and the second mode can be triggered depending on the phase in a gait cycle, the second mode being used *e.g.* during the stance phase of the gait cycle, and the first mode being used *e.g.* during the swing phase of the gait cycle. So, thanks to the torque controlling mechanism 300, and more particularly thanks to the clutch mechanism 340, motions of the prosthesis or orthosis are made more comfortable for the user and adaptable. More detailed embodiments of the clutch mechanism 340 are described below with reference to Figs.2A-2B, Figs.3A-3B, Figs.4A-4B, Figs.5A-5B, and Fig.6.

The second member 200 is pivotally coupled to the first member 100 around the first axis A1. The second member 200 depicted in Fig.1 comprises a sole element 230, and a linking element 220. The passive elastic element 210 is fixed to the second member 200, to a top front surface of the sole element 230 in the embodiment of Fig.1. The sole element 230 is configured for being in contact with the ground during a gait cycle. The linking element 220 is configured for linking the sole element 230 to the first member 100 such that the first member 100 is rotatable with respect to the linking element 220 around the first axis A1. In the embodiment of Fig.1, the linking element 220 has a U-shape with a first and second lateral portion 220a, 220b on either side of a central portion 220c. The linking element 220 is made of a rigid material. The first lateral portion 220a of the linking element is fixed to a top back surface of the sole element 230. The first axis A1 is located adjacently to the junctions between the central portion 220c and the second lateral portion 220b of the linking element.

In the embodiment of Fig.1, the clutch mechanism 340 is affixed to a static portion 311 of the motorized actuator 310. The static portion 311 of the motorized actuator 310 is pivotally coupled to the first member 100 around a first third axis A3'. Preferably, the first third axis A3' is parallel to the first axis A1. The torque controlling mechanism 300 comprises a translatable element 320 translating along the second axis A2. In the embodiment of Fig.1, the passive elastic element 210 is pivotally coupled to the translatable element 320 of the torque controlling mechanism 300 around the second third axis A3". Preferably, the second third axis A3" is parallel to the first axis A1 and the first third axis A3'.

By controlling rotation around the second axis A2, the clutch mechanism of Fig.1 can control the translation of the translatable element 320 along said second axis A2. In the embodiment of Fig.1, the torque controlling mechanism 300 is provided to the first member 100 such that the second axis A2 is substantially perpendicular to the first axis A1. Via the control of the translation of the translatable element 320, one can control a load application between the first member 100 and the passive elastic element 210. Through the control of the load application between the first member 100 and the passive elastic element 210, an indirect control of a rotation around the first axis A1 between the first member 100 and the second member 200 is achieved. In an alternative embodiment, the clutch mechanism 340 may be mounted around the first axis A1 to directly control the torque of the first member 100 with respect to the second member 200.

The passive elastic element 210 has a first extremity 211, here a rear extremity, and a second extremity 212, here a front extremity. The passive elastic element 210 is coupled to the second third axis A3" at the first extremity 211. The second third axis A3" is preferably parallel with the first axis A1. The passive elastic element 210 is fixed to the sole element 230 at the second extremity 212. The first extremity 211 and the second extremity 212 are located on either side of a vertical plane through the first axis A1 in the side view of Fig.1. When the translatable element 320 translates, a load is applied to the first extremity 211 of the passive elastic element. This load is transferred to the second extremity 212 of the passive elastic element fixed to the sole element 230. Due to the positioning of the first extremity 211 and the second extremity 212 relative to the first axis A1, a rotation of the second member 200 with respect to the first member 100 is provoked. The elasticity of the passive elastic element 210 allows attenuating abruptness of the rotation.

The passive elastic element 210 influences indirectly the rotation around the first axis A1 and is configured for storing energy when a load is applied to it, *e.g.* when the leg of the human subject presses the prosthesis against the ground. Indeed, when in the second mode of operation, as long as a load between the first member 100 and the passive elastic element 210 is exerted in the direction in which it is transmitted, the clutch mechanism 340 prevents translation of the translatable element 320.

However, rotation around the second third axis A3" and the first axis A1 is possible but met with the resistive nature of the passive elastic element 210. By increasing said torque, and thus a leveraging force applied to the first extremity 211 of the passive elastic element, above a predetermined resistive threshold, potential energy can be accumulated in the passive elastic element 210, and by decreasing said torque, the stored energy is again released as kinetic energy. Thus, an automatic adaptability of the prosthesis or orthosis to different operational conditions, such as walking on slopes or with varying speeds, can be obtained.

In another embodiment, the linking element may comprise a portion defining a top front of the foot linked to the sole element. The sole element and the linking element may be integrally formed and define a recess between the sole element and the linking element. The passive elastic element may be inserted within the recess and fixed to the portion of the linking element defining the top front of the foot.

In the embodiment of Fig.1, the torque controlling mechanism 300 further comprises a motorized actuator 310, preferably a ball screw actuator. In another embodiment, the torque controlling mechanism comprises a passive actuator instead. More particularly, the motorized actuator 310 comprises the first static portion 311 and a second dynamic portion (not shown). The second dynamic portion may rotate with respect to the first static portion around the second axis A2. The clutch mechanism 340 may control rotation of the second dynamic portion around the second axis A2. The second dynamic portion may be coupled to the translatable element 320 via a converting element (not shown). The converting element may convert the rotation of the second dynamic portion to a translation of the translatable element 320.

Figures 2A-2B shows perspective views of an exemplary embodiment of a clutch mechanism. The clutch mechanism 340 comprises: a base portion 341, a rotating member 347a, 347b, a one-way bearing 342, a shifter means 343, and a coupling mechanism 345. In an embodiment, the base portion 341 of the clutch mechanism may be fixed to an actuator of a torque controlling mechanism, *e.g.* the motorized actuator 310 of Fig.1.

The rotating member 347a, 347b is provided to the base portion 341, and is configured for rotating relative to the base portion 341 around a main axis A. In the embodiment of Fis.2A-2B, t he rotating member 347a, 347b comprises a cylindrical portion 347b extending along the main axis A. In the embodiment of Figs.2A-2B, the rotating member 347a, 347b also comprises a crown portion 347a extending substantially perpendicularly to the main axis A.

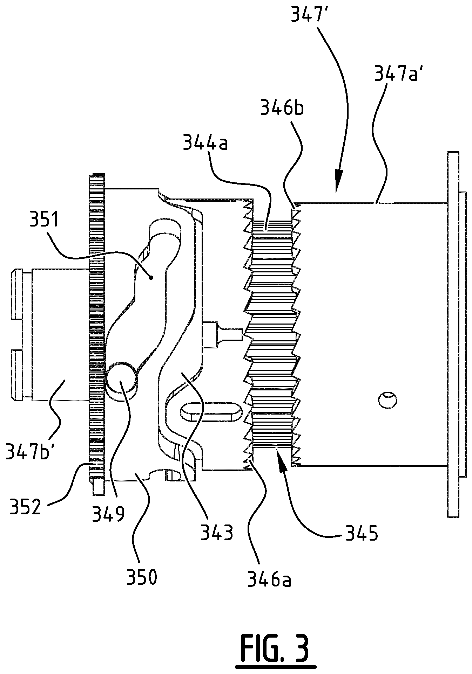

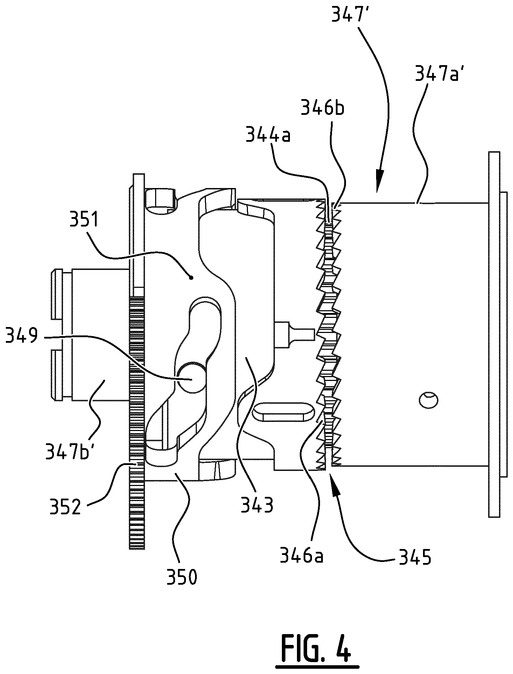

The one-way bearing 342 is provided to the rotating member along the main axis A, and more specifically is arranged around the cylindrical portion 347b of the rotating member. The shifter means 343 is configured for being translatable along the main axis A and for being non-rotatable relative to the base portion 341. The coupling mechanism 345 is fixed around the one-way bearing 342, and is configured for having a first position coupled to the shifter means 343, as further detailed with reference to Figs.4A-4B below, corresponding to a second mode of operation of the clutch mechanism 340, and a second position uncoupled from the shifter means 343, as further detailed with reference to Figs.3A-3B below, corresponding to a first mode of operation of the clutch mechanism. A rotation of the coupling mechanism 345 around the main axis A relative to the base portion 341 is prevented in the first position and is allowed in the second position.

In the embodiments of Figs.2A-2B, the different elements of the clutch mechanism 340 are arranged in a concentric manner around the main axis A, which ensures an overall compact form factor of the clutch mechanism 340. In an embodiment, the main axis A may coincide with the axis or rotation between a first member and a second member which are pivotally coupled, *e.g.* the first axis A1 in Fig.1, and the clutch mechanism 340 influences directly the rotation of the first member relative to the second member at the level of their pivot coupling. In another embodiment, the main axis A may be perpendicular to said axis of rotation and a converting means may be provided between the rotating member 347a, 347b and one of the first member or the second member to convert a rotation around the main axis to a rotation around said axis of rotation.

The coupling mechanism 345 comprises a first set of gear teeth 344a on its periphery, and the shifter means 343 comprises a second set of gear teeth 344b complementary to the first set of gear teeth 344a. At least one of the first set 344a and the second set of gear teeth 344b, both sets in the embodiment of Figs.2A-2B, has gear teeth with a sloped face facing the gear teeth of the other one of the first set 344a or the second set of gear teeth 344b such as to promote engagement of the first set 344a and second set of gear teeth 344b when transitioning from the second position to the first position of the coupling mechanism 345.

The shifter means 343 is arranged over the coupling mechanism 345 in a coupled state. The coupling mechanism 345 comprises a hollow body, preferably a cylindrical hollow body, having an outer surface. The shifter means 343 comprises a hollow body, preferably a cylindrical hollow body, having an inner surface. The first set of gear teeth 344a is provided to the outer surface of the coupling mechanism 345, and the second set of gear teeth 344b is provided to the inner surface of the shifter means 343.

The clutch mechanism 340 further comprises a blocking means including a first blocking part 346a configured for inter-engaging with a second blocking part 346b, said first blocking part 346a fixed to the shifter means 343 and said second blocking part 346b fixed to the rotating member 346b. The blocking means 346a, 346b is further configured for, in an inter-engaged state of the first blocking part 346a and the second blocking part 346b, preventing the rotation of the rotating member 347a, 347b around the main axis A allowed by the one-way bearing 342. This inter-engaged state corresponds to a third mode wherein rotation around the main axis A is blocked in both directions.

In the embodiments of Figs.2A-2B, the first blocking part 346a and the second blocking part 346b comprises each a complementary pair of inter-engaging teeth arranged in a concentric manner around the main axis A. More particularly, the shifter means 343 may comprise a circular row of saw teeth 346a and the crown portion 347b of the rotating member may comprise a corresponding row of saw teeth 346b. In the embodiment of Figs.2A-2B, the circular row of saw teeth 344b may be arranged on an edge of the cylindrical body of the shifter means 343.

When transitioning from the first mode to the second mode, the shifter means 343 and the coupling mechanism 345 are configured to couple, thereby allowing rotation around the main axis A in a direction dependent on the one-way bearing 342. When transitioning from the second mode to the third mode, the shifter means 343 and the coupling mechanism 345 are still coupled, but the shifter means 343 further engages with the rotating member 347a, 347b at the level of the first 346a and second blocking parts 346b, therefore preventing rotation around the main axis A.

The shifter means 343 is configured for being translatable along the main axis A. In the embodiments of Figs.2A-2B, the clutch mechanism 340 comprises a first and a second stopping means 348a, 348b cooperating with each other. The first stopping means 348a is provided to the shifter means 343. More particularly, the first stopping means 348a comprises a plurality of ears extending substantially perpendicularly to the main axis A. The second stopping means 348b is provided to the base portion 341 of the clutch mechanism. The second stopping means 348b comprises a corresponding plurality of channels extending along the main axis A. When the clutch mechanism 340 is assembled, the first stopping means 348a is slotted within the second stopping means 348b. Thus, rotation of the shifter means 343 around the main axis A relative to the base portion 341 is prevented while translation along the main axis A is allowed.

The clutch mechanism 340 may comprise a selecting means 350. In the embodiments of Figs.2A-2B, the selecting means 350 is rotatable and configured for selecting one of the at least three modes of operations by transitioning between the first mode and the second mode, and between the second mode and the third mode. In this manner, rotation of the first member relative to the second member is controllable at will. The selecting means 350 may be used to transition between the different modes at different timings depending on the operational conditions, *e.g*. walking, running, flat surface, incline, and decline.

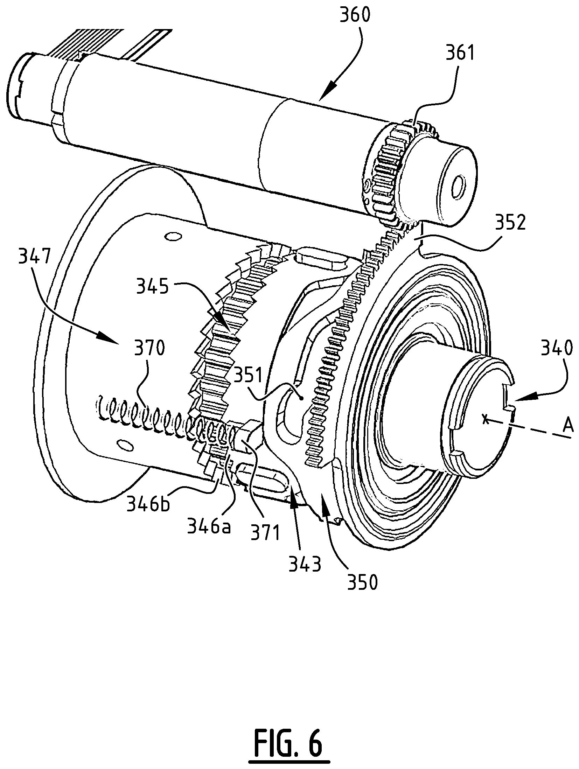

In an embodiment, the selecting means 350 can be controlled automatically following a control scheme implemented using a multimodal motorized actuator 353. For example, the prosthesis or orthosis may comprise a microcontroller configured for controlling the multimodal motorized actuator, and a memory for storing a plurality of control schemes depending on the operational conditions. The microcontroller may control the multimodal motorized actuator 353 such that the clutch mechanism 340 transitions between the different modes of operations following the predetermined control scheme. In the embodiments of Figs.2A-2B, the selecting means 350 may be rotated into a plurality of positions in a continuous way via gear portion 352, a more detailed embodiment is described below with reference to Fig.6.

The selecting means 350 of Figs.2A-2B, is provided to the shifter means 343 and is configured for converting a rotation of the selecting means 350 into a translation of the shifter means 343. The selecting means 350 may be rotated around the main axis A.

More particularly, the shifter means 343 may comprise at least one protruding portion 349, *e.g*. comprising at least one rolling ball. The selecting means 350 may comprise a selector ring surrounding the shifter means 343. The selector ring comprises at least one cut-out portion 351 configured for cooperating with the at least one protruding portion 349 of the shifter means 343, such that the rotation of the selecting means 350 is converted into the translation of the shifter means 343 by the cooperation of the at least one cut-out portion 351 and the at least one protruding portion 349.

The selector ring of the selecting means 350 may extend along the main axis A and may be centered around the main axis A. The at least one protruding portion 349 may be fixed on an outer surface of the shifter means 343. The selector ring of the selecting means 350 may be arranged around the shifter means 343 such that the at least one protruding portion 349 is inserted in the at least one cut-out portion 351. Depending on the profile of the at least one cut-out portion 351, the at least one protruding portion 349 may be pushed into different positions, thus provoking the translation of the shifter means 343.

Each of the at least one cut-out portion 351 may comprise a plurality of areas. The plurality of areas may include transitioning areas and mode areas. The mode areas can correspond to stable positions of the shifter means 343 with respect to the coupling mechanism 345 and/or the rotating member 347 such that the clutch mechanism 340 is in one of the at least three operation modes. The transitioning areas may comprise profiles sized to correspond to the travel distance necessary for the shifter means 343 to translate such that a smooth transition is achieved between the first mode and the second mode, the second mode and the third mode, and *vice versa*.

In addition to the first mode and the second mode, optionally also in addition to the third mode, the clutch mechanism 340 may be further configured for controlling the rotation around the main axis A in at least a fourth mode of operation, in which energy is stored in at least one spring 370 to trigger a change from the second mode to the first mode when the energy stored in the at least one spring 370 is under a threshold. This fourth mode can be defined as a "floating" mode of operation located between the first mode and the second mode whose transition from the second mode is triggered by the selecting means 350, and whose transition to the first mode is mechanically conditioned.

In the embodiment of Figs.2A-2B, the at least one spring 370 is mounted between the shifter means 343 and the base portion 341. Preferably, at least three springs 370 are mounted at regular intervals around the main axis A. The at least one spring 370 may be fixed to a peripheral protruding portion of the shifter means 343, here fixed to the first stopping means 348a. By being fixed between the shifter means 343, which moves mainly in translation, and the base portion 341, the spring force is exerted mainly along the translation direction of the shifter means 343. At the same time, slight rotation motions of the shifter means 343 around the main axis A which could occur during coupling and uncoupling of the shifter means 343 and the coupling mechanism 345 are still allowed. Preferably, the at least one spring 370 comprises at least one helical tension springs.

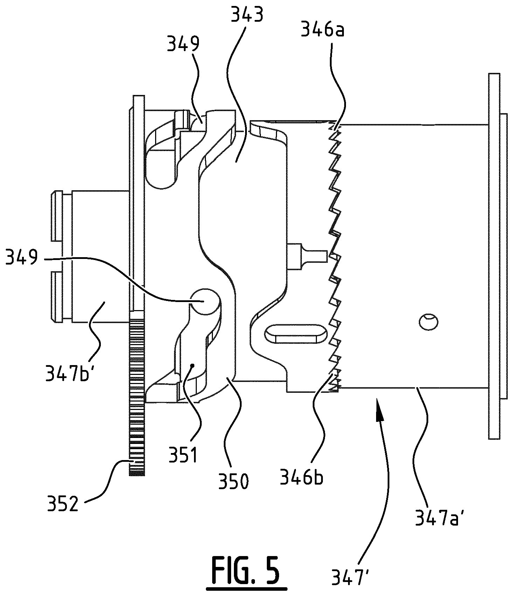

Figure 3 depicts a perspective view of an exemplary embodiment of a first mode of operation of the clutch mechanism. Figure 4 depicts a perspective view of an exemplary embodiment of a second mode of operation of the clutch mechanism. Figure 5 depicts a perspective view of an exemplary embodiment of a third mode of operation of the clutch mechanism. Embodiments of the clutch mechanism 340 depicted in Fig.3, Fig.4, and Fig.5 are similar to the one described with respect to Figs.2A-2B.

In Fig.3, the selecting means 350 maintains the shifter means 343 in the first mode of operation. The shifter means 343 is uncoupled from the coupling mechanism 345. So, the coupling mechanism 345 may rotate in both directions with respect to the shifter means 343 around the main axis A. The one-way bearing (not shown) may allow rotation around the main axis A in a clockwise direction and prevent rotation in an anti-clockwise direction. The cylindrical portion 347b' of the rotating member 347' being fixed to the one-way bearing, and the one-way bearing being fixed to the coupling mechanism 345, the rotating member 347' can rotate freely clockwise around the main axis A due to the allowed rotation of the one-way bearing, and can rotate freely anti-clockwise around the main axis A due to the allowed rotation of the coupling mechanism 345 relative to the shifter means 343.

In Fig.4, the selecting means 350 maintains the shifter means 343 in the second mode of operation. The shifter means 343 has been translated over the first set of gear teeth 344a. The shifter means 343 and the coupling mechanism 345 are therefore coupled. The rotating member 347' can rotate still freely clockwise around the main axis A due to the allowed rotation of the one-way bearing, but cannot rotate anti-clockwise anymore around the main axis A due to the fixed coupling between the coupling mechanism 345 and the shifter means 343.

In Fig.5, the selecting means 350 maintains the shifter means 343 in the third mode of operation. The shifter means 343 and the coupling mechanism 345 are still coupled. Additionally, the first blocking part 346a of the shifter means 343 is inter-engaged with the second blocking part 346b of the crown portion 347a'. Anti-clockwise rotation around the main axis A is still prevented due to the fixed coupling between the coupling mechanism 345 and the shifter means 343, and clockwise rotation around the main axis A is also prevented due to the rotating member 347' and the shifter means 343 being inter-engaged.

Figure 6 illustrates a close-up view of another exemplary embodiment of a clutch mechanism. The embodiment of the clutch mechanism 340 depicted in Fig.6 is similar to the one described with reference to Figs.2A-2B, 3, 4, and 5.

In the embodiment of Fig.6, the prosthesis or orthosis may comprise a microcontroller configured for controlling a multimodal motorized actuator 360, and a memory for storing a plurality of control schemes depending on operational conditions of the prosthesis or orthosis. The microcontroller may control the multimodal motorized actuator 360 such that the clutch mechanism 340 transitions between the different modes of operation following the predetermined control scheme.

The selecting means 350 may be rotated into a plurality of positions in a continuous way via gear portion 352 of the selecting means 350. The gear portion 352 may rotate about main axis A. Rotation of the gear portion 352 may be caused by a geared circular element 361 of the multimodal motorized actuator 360 engaged with the gear portion 352.

In addition to the first mode and the second mode, optionally also in addition to the third mode, the clutch mechanism 340 may be further configured for controlling the rotation around the main axis A in at least a fourth mode of operation, in which energy is stored in at least one spring 370 to trigger a change from the second mode to the first mode when the energy stored in the spring 370 is under a threshold. This fourth mode can be defined as a "floating" mode of operation located between the first mode and the second mode whose transition from the second mode is triggered by the selecting means, and whose transition to the first mode is mechanically conditioned.

In the embodiment of Fig.6, the at least one spring 370 is mounted between the shifter means 343 and the base portion (not shown). The at least one spring 370 may be fixed to a peripheral protruding portion 371 of the shifter means 343. By being fixed between the shifter means, which moves mainly in translation, and the base portion, the spring force is exerted mainly along the translation direction of the shifter means 343. At the same time, slight rotation motions of the shifter means 343 around the main axis A which could occur during coupling and uncoupling of the shifter means 343 and the coupling mechanism 345 are still allowed. Preferably, the spring 370 is a helical tension spring.

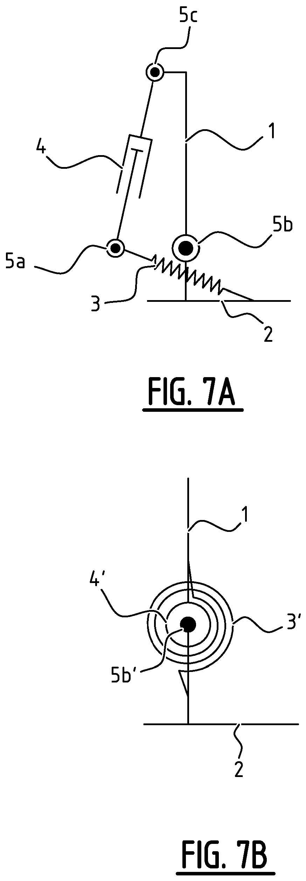

Figures 7A-7B show schematic structural embodiments of a prosthesis or orthosis according to the present invention. Depending on the figures, the prosthesis or orthosis comprises a first member 1, a second member 2, a passive elastic element 3, 3', and a torque controlling mechanism 4, 4'. The torque controlling mechanism 4, 4' is configured for controlling a torque around a first axis between the first member 1 and the second member 2 . The torque controlling mechanism 4, 4' comprises a clutch mechanism, and optionally a motorized actuator. The clutch mechanism is configured for controlling a rotation around its main axis of rotation. The torque controlling mechanism 4, 4' is configured for controlling the torque around the first axis in at least two modes of operations:
- a first mode wherein load is not transmitted between the first member 1 and the passive elastic element 3, 3' in both working directions of the passive elastic element 3, 3',
- a second mode wherein load is transmitted between the first member 1 and the passive elastic element 3, 3' in one working direction of the passive elastic element 3, 3' and not transmitted impeded in the other working direction of the passive elastic element 3, 3'.

As can be seen in Figs.7A-7B, the different elements of the prosthesis or orthosis are coupled together via pivot joints 5a, 5b, 5c, 5b' allowing rotation between the coupled elements.

In the embodiment of Fig.7A, a first extremity of the passive elastic element 3 is fixed to a front part of the second member 2 and coupled at its second extremity to a first end of the torque controlling mechanism 4 via a first pivot joint 5a. The passive elastic element 3 is configured to work in the plane of the schematic. The torque controlling mechanism 4 is configured to control a translating motion between its first and second end. The first member 1 and the second member 2 are coupled via a second pivot joint 5b. The first member 1 is also coupled to the second end of the torque controlling mechanism 4 via a third pivot joint 5c.

In the embodiment of Fig.7B, the first member 1 is coupled to the second member via another second pivot joint 5b'. Another passive elastic element 3' is fixed between the first member 1 and the second member 2, and configured to work in torsion around the same axis as the rotation axis of the another second pivot joint 5b'. Another torque controlling mechanism 4' is arranged to control a torque between the first member 1 and the second member 2. The another passive elastic element 3' acts on a rotation motion around the rotation axis of the another second rotating joint 5b', and the torque is controlled by the torque controlling mechanism 4' around a coaxial axis.

In light of the above, the skilled person will understand that the organization and coupling of the different elements of the prosthesis or orthosis can be structured in many ways while including the passive elastic element and allowing the torque controlling mechanism to control the rotation of the first member with respect to the second member.

Whilst the principles of the invention have been set out above in connection with specific embodiments, it is to be understood that this description is merely made by way of example and not as a limitation of the scope of protection which is determined by the appended claims.

## Claims

1. A prosthesis or orthosis comprising a hinge joint system for functionally assisting, enhancing, and/or replacing a hinge joint of a human or animal subject, said hinge joint system comprising:
- a first member (100);
- a second member (200) pivotally coupled to the first member around a first axis (A1);
- a passive elastic element (210) fixed to the second member and acting on the rotation around the first axis, and which is configured for storing energy when a load is applied to said passive elastic element;
- a torque controlling mechanism (300) mounted between the first member and the passive elastic element;
**characterized in that** the torque controlling mechanism is configured for controlling a torque around the first axis in at least three modes of operations:
- a first mode wherein load is not transmitted between the first member and the passive elastic element in both working directions of the passive elastic element,
- a second mode wherein load is transmitted between the first member and the passive elastic element in one working direction of the passive elastic element and not transmitted in the other working direction of the passive elastic element,
- a third mode wherein load is transmitted between the first member and the passive elastic element in both working directions of the passive elastic element.

2. The prosthesis or orthosis of claim 1, wherein the torque controlling mechanism comprises a selecting means, preferably a rotatable selecting means, configured for selecting one of the at least three modes of operations.

3. The prosthesis or orthosis of claim 2, wherein the torque controlling mechanism further comprises a first motorized actuator configured for actuating the selecting means.

4. The prosthesis or orthosis of any one of the previous claims, wherein the torque controlling mechanism is further configured for controlling the torque around the first axis in at least a fourth mode of operation, in which energy is stored, preferably in at least one spring, to trigger a change from the second mode to the first mode when the stored energy is under a threshold.

5. The prosthesis or orthosis of any one of the previous claims, wherein the torque controlling mechanism further comprises a stopper element configured for stopping the load from being applied to the passive elastic element in one working direction.

6. The prosthesis or orthosis of any one of the previous claims, wherein the torque controlling mechanism comprises a clutch mechanism, said clutch mechanism comprising:
- a base portion;
- a rotating member provided to the base portion, said rotating member configured for rotating relative to the base portion around a second axis;
- a one-way bearing provided to the rotating member along the second axis;
- a shifter means configured for being translatable along the second axis and substantially non-rotatable relative to the base portion;
- a coupling mechanism fixed around the one-way bearing and configured for having a first position coupled to the shifter means and a second position uncoupled from the shifter means, such that a rotation of the coupling mechanism around the second axis relative to the base portion is prevented in the first position and allowed in the second position.

7. The prosthesis or orthosis of claims 2 and 6, wherein the selecting means is provided to the shifter means and configured for converting a rotation of the selecting means into a translation of the shifter means.

8. The prosthesis or orthosis of claims 4 and 7, wherein the at least one spring is mounted between the shifter means and the base portion.

9. The prosthesis or orthosis of any one of claims 6-8, wherein the torque controlling mechanism further comprises a second motorized actuator configured for rotating the rotating member of the torque controlling mechanism; and, optionally, wherein the clutch mechanism is mounted to the second motorized actuator.

10. The prosthesis or orthosis of any one of claims 6-9, wherein the coupling mechanism comprises a first set of gear teeth on its periphery, and the shifter means comprises a second set of gear teeth complementary to the first set of gear teeth; wherein at least one of the first set or the second set of gear teeth has gear teeth with a sloped face facing the gear teeth of the other one of the first set or the second set of gear teeth such as to promote engagement of the first set and second set of gear teeth when transitioning from the second position to the first position of the coupling mechanism.

11. The prosthesis or orthosis of claim 6, wherein the clutch mechanism further comprises a blocking means including a first blocking part configured for inter-engaging with a second blocking part, said first blocking part fixed to the shifter means and said second blocking part fixed to the rotating member; wherein the blocking means is further configured for, in an inter-engaged state of the first blocking part and the second blocking part, preventing the rotation of the rotating member around the second axis allowed by the one-way bearing.

12. The prosthesis or orthosis of claims 2 and 6, wherein the selecting means comprises a selector ring surrounding the shifter means; wherein the selector ring comprises at least one cut-out portion configured for cooperating with at least one corresponding protruding portion of the shifter means, such that the rotation of the selecting means is converted into the translation of the shifter means by the cooperation of the at least one cut-out portion and the at least one corresponding protruding portion.

13. The prosthesis or orthosis of the previous claim, wherein the at least one corresponding protruding portion comprises at least one rolling ball.

14. The prosthesis or orthosis of any one of the previous claims, wherein the torque controlling mechanism is further configured for controlling the motion of the first member with respect to the second member through leverage.

15. The prosthesis or orthosis of claim 6, wherein the torque controlling mechanism further comprises a converting means configured for interconnecting the rotating member and one of the first member or the second member, and for converting the rotation of the rotating member around the second axis to a rotation around a third axis and vice versa, said third axis being preferably parallel to the first axis.

## Patentansprüche

1. Prothese oder Orthese, umfassend ein Winkelgelenksystem zum funktionellen Unterstützen, Verbessern und/oder Ersetzen eines Winkelgelenks eines menschlichen oder tierischen Subjekts, das Winkelgelenksystem umfassend:
- ein erstes Glied (100);
- ein zweites Glied (200), das mit dem ersten Glied um eine erste Achse (A1) herum schwenkbar gekoppelt ist;
- ein passives elastisches Element (210), das an dem zweiten Glied befestigt ist und auf die Drehung um die erste Achse herum einwirkt und das zum Speichern von Energie konfiguriert ist, wenn eine Last auf das passive elastische Element ausgeübt wird;
- einen Drehmomentsteuermechanismus (300), der zwischen dem ersten Glied und dem passiven elastischen Element montiert ist;
**dadurch gekennzeichnet, dass** der Drehmomentsteuermechanismus zum Steuern eines Drehmoments um die erste Achse herum in mindestens drei Betriebsmodi konfiguriert ist:
- einem ersten Modus, wobei die Last zwischen dem ersten Glied und dem passiven elastischen Element in beiden Arbeitsrichtungen des passiven elastischen Elements nicht übertragen wird,
- einem zweiten Modus, wobei die Last zwischen dem ersten Glied und dem passiven elastischen Element in einer Arbeitsrichtung des passiven elastischen Elements übertragen wird und in der anderen Arbeitsrichtung des passiven elastischen Elements nicht übertragen wird,
- einem dritten Modus, wobei die Last zwischen dem ersten Glied und dem passiven elastischen Element in beiden Arbeitsrichtungen des passiven elastischen Elements übertragen wird.

2. Prothese oder Orthese nach Anspruch 1, wobei der Drehmomentsteuermechanismus ein Auswahlmittel, vorzugsweise ein drehbares Auswahlmittel, umfasst, das zum Auswählen eines der mindestens drei Betriebsmodi konfiguriert ist.

3. Prothese oder Orthese nach Anspruch 2, wobei der Drehmomentsteuermechanismus ferner ein erstes motorisiertes Betätigungselement umfasst, das zum Betätigen des Auswahlmittels konfiguriert ist.

4. Prothese oder Orthese nach einem der vorstehenden Ansprüche, wobei der Drehmomentsteuermechanismus ferner zum Steuern des Drehmoments um die erste Achse herum in mindestens einem vierten Betriebsmodus konfiguriert ist, in dem Energie gespeichert wird, vorzugsweise in mindestens einer Feder, um einen Wechsel von dem zweiten Modus in den ersten Modus auszulösen, wenn die gespeicherte Energie unter einer Schwelle liegt.

5. Prothese oder Orthese nach einem der vorstehenden Ansprüche, wobei der Drehmomentsteuermechanismus ferner ein Anschlagelement umfasst, das konfiguriert ist, um die Last abzuhalten, auf das passive elastische Element in einer Arbeitsrichtung aufgebracht zu werden.

6. Prothese oder Orthese nach einem der vorstehenden Ansprüche, wobei der Drehmomentsteuermechanismus einen Kupplungsmechanismus umfasst, der Kupplungsmechanismus umfassend:
- einen Basisabschnitt;
- ein drehbares Glied, das dem Basisabschnitt bereitgestellt ist, wobei das drehbare Glied zum Drehen relativ zu dem Basisabschnitt um eine zweite Achse herum konfiguriert ist;
- ein Einweglager, das dem drehbaren Glied entlang der zweiten Achse bereitgestellt ist;
- ein Schaltmittel, das konfiguriert ist, um entlang der zweiten Achse verlagerungsfähig und relativ zu dem Basisabschnitt im Wesentlichen nicht drehbar zu sein;
- einen Kopplungsmechanismus, der um das Einweglager herum befestigt und zum Aufweisen einer ersten Position, die mit dem Schaltmittel gekoppelt ist, und einer zweiten Position, die von dem Schaltmittel entkoppelt ist, konfiguriert ist, derart dass eine Drehung des Kopplungsmechanismus um die zweite Achse herum relativ zu dem Basisabschnitt in der ersten Position verhindert und in der zweiten Position zugelassen wird.

7. Prothese oder Orthese nach den Ansprüchen 2 und 6, wobei das Auswahlmittel dem Schaltmittel bereitgestellt ist und zum Umwandeln einer Drehung des Auswahlmittels in eine Verschiebung des Schaltmittels konfiguriert ist.

8. Prothese oder Orthese nach den Ansprüchen 4 und 7, wobei die mindestens eine Feder zwischen dem Schaltmittel und dem Basisabschnitt montiert ist.

9. Prothese oder Orthese nach einem der Ansprüche 6 bis 8, wobei der Drehmomentsteuermechanismus ferner ein zweites motorisiertes Betätigungselement umfasst, das zum Drehen des drehbaren Glieds des Drehmomentsteuermechanismus konfiguriert ist; und, optional, wobei der Kupplungsmechanismus an dem zweiten motorisierten Betätigungselement montiert ist.

10. Prothese oder Orthese nach einem der Ansprüche 6 bis 9, wobei der Kopplungsmechanismus einen ersten Satz von Zahnradzähnen an seinem Umfang umfasst und das Schaltmittel einen zweiten Satz von Zahnradzähnen umfasst, der komplementär zu dem ersten Satz von Zahnradzähnen ist; wobei mindestens einer des ersten Satzes oder des zweiten Satzes von Zahnradzähnen Zahnradzähne mit einer geneigten Fläche aufweist, die den Zahnradzähnen des anderen des ersten Satzes oder des zweiten Satzes von Zahnradzähnen zugewandt ist, wie, um einen Eingriff des ersten Satzes und des zweiten Satzes von Zahnradzähnen bei einem Übergehen von der zweiten Position in die erste Position des Kopplungsmechanismus zu fördern.

11. Prothese oder Orthese nach Anspruch 6, wobei der Kupplungsmechanismus ferner ein Blockiermittel umfasst, das ein erstes Blockierteil einschließt, das zum Ineinandereingreifen mit einem zweiten Blockierteil konfiguriert ist, wobei das erste Blockierteil an dem Schaltmittel befestigt ist und das zweite Blockierteil an dem drehbaren Glied befestigt ist; wobei das Blockiermittel ferner, in einem ineinandereingreifenden Zustand des ersten Blockierteils und des zweiten Blockierteils, zum Verhindern der Drehung des drehbaren Glieds um die zweite Achse herum konfiguriert ist, die durch das Einweglager zugelassen wird.

12. Prothese oder Orthese nach den Ansprüchen 2 und 6, wobei das Auswahlmittel einen Auswahlring umfasst, der das Schaltmittel umgibt; wobei der Auswahlring mindestens einen Aussparungsabschnitt umfasst, der zum derartigen Zusammenwirken mit mindestens einem entsprechenden vorstehenden Abschnitt des Schaltmittels konfiguriert ist, dass die Drehung des Auswahlmittels durch das Zusammenwirken des mindestens einen Aussparungsabschnitts und des mindestens einen entsprechenden vorstehenden Abschnitts in die Verschiebung des Schaltmittels umgewandelt wird.

13. Prothese oder Orthese nach dem vorstehenden Anspruch, wobei der mindestens eine entsprechende vorstehende Abschnitt mindestens eine Rollkugel umfasst.

14. Prothese oder Orthese nach einem der vorstehenden Ansprüche, wobei der Drehmomentsteuermechanismus ferner zum Steuern der Bewegung des ersten Glieds in Bezug auf das zweite Glied durch eine Hebelwirkung konfiguriert ist.

15. Prothese oder Orthese nach Anspruch 6, wobei der Drehmomentsteuermechanismus ferner ein Umwandlungsmittel umfasst, das zum Verbinden des drehbaren Glieds und eines von dem ersten Glied oder dem zweiten Glied und zum Umwandeln der Drehung des drehbaren Glieds um die zweite Achse herum in eine Drehung um eine dritte Achse herum und umgekehrt konfiguriert ist, wobei die dritte Achse vorzugsweise parallel zu der ersten Achse ist.

## Revendications

1. Prothèse ou orthèse comprenant un système d'articulation à charnière permettant d'assister, d'améliorer, et/ou de remplacer fonctionnellement une articulation à charnière d'un sujet humain ou animal, ledit système d'articulation à charnière comprenant :
- un premier élément (100) ;
- un second élément (200) accouplé de manière pivotante au premier élément autour d'un premier axe (A1) ;
- un élément élastique passif (210) fixé au second élément et agissant sur la rotation autour du premier axe, et qui est conçu pour stocker de l'énergie lorsqu'une charge est appliquée audit élément élastique passif ;
- un mécanisme de commande de couple (300) monté entre le premier élément et l'élément élastique passif ;
**caractérisé en ce que** le mécanisme de régulation de couple est conçu pour réguler un couple autour du premier axe dans au moins trois modes de fonctionnement :
- un premier mode dans lequel la charge n'est pas transmise entre le premier élément et l'élément élastique passif dans les deux sens de travail de l'élément élastique passif,
- un deuxième mode dans lequel la charge est transmise entre le premier élément et l'élément élastique passif dans un sens de travail de l'élément élastique passif et non transmise dans l'autre sens de travail de l'élément élastique passif,
- un troisième mode dans lequel la charge est transmise entre le premier élément et l'élément élastique passif dans les deux sens de travail de l'élément élastique passif.

2. Prothèse ou orthèse selon la revendication 1, dans laquelle le mécanisme de commande de couple comprend un moyen de sélection, de préférence un moyen de sélection rotatif, conçu pour sélectionner l'un des au moins trois modes de fonctionnement.

3. Prothèse ou orthèse selon la revendication 2, dans laquelle le mécanisme de commande de couple comprend en outre un premier actionneur motorisé conçu pour actionner le moyen de sélection.

4. Prothèse ou orthèse selon l'une quelconque des revendications précédentes, dans laquelle le mécanisme de commande de couple est en outre configuré pour commander le couple autour du premier axe dans au moins un quatrième mode de fonctionnement, dans lequel l'énergie est stockée, de préférence dans au moins un ressort, pour déclencher un changement du deuxième mode au premier mode lorsque l'énergie stockée est inférieure à un seuil.

5. Prothèse ou orthèse selon l'une quelconque des revendications précédentes, dans laquelle le mécanisme de commande de couple comprend en outre un élément d'arrêt conçu pour arrêter l'application de la charge à l'élément élastique passif dans une direction de travail.

6. Prothèse ou orthèse selon l'une quelconque des revendications précédentes, dans laquelle le mécanisme de commande de couple comprend un mécanisme d'embrayage, ledit mécanisme d'embrayage comprenant :
- une partie de base ;
- un élément rotatif fourni à la partie de base, ledit élément rotatif étant conçu pour tourner par rapport à la partie de base autour d'un second axe ;
- un palier unidirectionnel fourni à l'élément rotatif le long du second axe ;
- un moyen de déplacement conçu pour être mobile en translation le long du second axe et sensiblement non rotatif par rapport à la partie de base ;
- un mécanisme d'accouplement fixé autour du palier unidirectionnel et conçu pour avoir une première position accouplée au moyen de changement de vitesse et une seconde position découplée du moyen de changement de vitesse, de sorte qu'une rotation du mécanisme d'accouplement autour du second axe par rapport à la partie de base est empêchée dans la première position et permise dans la seconde position.

7. Prothèse ou orthèse selon les revendications 2 et 6, dans laquelle le moyen de sélection est fourni au moyen de déplacement et conçu pour convertir une rotation du moyen de sélection en une translation du moyen de déplacement.

8. Prothèse ou orthèse selon les revendications 4 et 7, dans laquelle l'au moins un ressort est monté entre le moyen de déplacement et la partie de base.

9. Prothèse ou orthèse selon l'une quelconque des revendications 6 à 8, dans laquelle le mécanisme de commande de couple comprend en outre un second actionneur motorisé conçu pour faire tourner l'élément rotatif du mécanisme de commande de couple ; et, éventuellement, dans lequel le mécanisme d'embrayage est monté sur le second actionneur motorisé.

10. Prothèse ou orthèse selon l'une quelconque des revendications 6 à 9, dans laquelle le mécanisme d'accouplement comprend un premier ensemble de dents d'engrenage sur sa périphérie, et le moyen de changement de vitesse comprend un second ensemble de dents d'engrenage complémentaire au premier ensemble de dents d'engrenage ; dans lequel au moins l'une du premier ensemble ou du second ensemble de dents d'engrenage a des dents d'engrenage avec une face inclinée faisant face aux dents d'engrenage de l'autre du premier ensemble ou du second ensemble de dents d'engrenage de manière à promouvoir la mise en prise du premier ensemble et du second ensemble de dents d'engrenage lors de la transition de la seconde position à la première position du mécanisme d'accouplement.

11. Prothèse ou orthèse selon la revendication 6, dans laquelle le mécanisme d'embrayage comprend en outre un moyen de blocage comportant une première partie de blocage conçue pour s'inter-engager avec une seconde partie de blocage, ladite première partie de blocage étant fixée au moyen de changement de vitesse et ladite seconde partie de blocage étant fixée à l'élément rotatif ; dans lequel le moyen de blocage est en outre conçu pour, dans un état de mise en prise de la première partie de blocage et de la seconde partie de blocage, empêcher la rotation de l'élément rotatif autour du second axe permise par le palier unidirectionnel.

12. Prothèse ou orthèse selon les revendications 2 et 6, dans laquelle le moyen de sélection comprend un anneau de sélection entourant le moyen de déplacement ; dans lequel l'anneau de sélection comprend au moins une partie de découpe conçue pour coopérer avec au moins une partie saillante correspondante du moyen de déplacement de vitesse, de telle sorte que la rotation du moyen de sélection est convertie en translation du moyen de déplacement par la coopération de l'au moins une partie de découpe et de l'au moins une partie saillante correspondante.

13. Prothèse ou orthèse selon la revendication précédente, dans laquelle l'au moins une partie saillante correspondante comprend au moins une bille roulante.

14. Prothèse ou orthèse selon l'une quelconque des revendications précédentes, dans laquelle le mécanisme de commande de couple est en outre conçu pour commander le mouvement du premier élément par rapport au second élément par l'intermédiaire d'un effet de levier.

15. Prothèse ou orthèse selon la revendication 6, dans laquelle le mécanisme de commande de couple comprend en outre un moyen de conversion conçu pour inter-relier l'élément rotatif et l'un du premier élément ou du second élément, et pour convertir la rotation de l'élément rotatif autour du deuxième axe en une rotation autour d'un troisième axe et vice versa, ledit troisième axe étant de préférence parallèle au premier axe.
